# EUROPÄISCHE PATENTSCHRIFT

(11) **EP 1 349 582 B1**
(45) Veröffentlichungstag und Bekanntmachung des Hinweises auf die Patenterteilung: **24.11.2004**
(21) Anmeldenummer: 02715415.2
(22) Anmeldetag: 11.01.2002
(51) Int. Cl.: A61L 27/34, C08G 79/02, C12N 5/00

(54) **POLYPHOSPHAZEN-HALTIGE SUBSTRATE ALS MATRIZE FÜR DIE ZÜCHTUNG VON ZELLEN**
SUBSTRATES CONTAINING POLYPHOSPHAZENE AS MATRIX FOR CELL CULTURE
SUBSTRATS CONTENANT DES POLYPHOSPHAZENES SERVANT DE MATRICE POUR LA CULTURE DE CELLULES

(30) Priorität: 11.01.2001 DE 10100961
(43) Veröffentlichungstag der Anmeldung: 08.10.2003
(60) Teilanmeldung: 04020856.3
(73) Patentinhaber: Polyzenix GmbH, 89077 Ulm (DE)
(72) Erfinder: GRUNZE, Michael, 69151 Neckargemünd (DE)
(74) Vertreter: Müller-Boré & Partner Patentanwälte
(86) Internationale Anmeldenummer: PCT/EP2002/000230
(87) Internationale Veröffentlichungsnummer: WO 2002/064666

(56) Entgegenhaltungen:
- WO-A-01/80919
- WO-A-99/45096
- DE-A- 19 613 048
- LAURENCIN C.T. ET AL.: "Use of polyphosphazenes for skeletal tissue regeneration" JOURNAL OF BIOMEDICAL MATERIALS RESEARCH, Bd. 27, Nr. 7, 1993, Seiten 963-973, XP008004104

## Beschreibung

Die vorliegende Erfindung betrifft Polyphosphazen-haltige Substrate mit einer formgebenden Oberfläche als Matrize zur Herstellung von in einen Säuger implantierbarer biologischer Materialien, Verfahren zur Herstellung derartiger Substrate sowie Polyphosphazen-haltige Substrate mit mikrostrukturierter Oberfläche.

Die Züchtung von Zellen, insbesondere von Endothelzellen, mit dem Ziel künstliche Organe wachsen zu lassen, ist eine vergleichsweise neue Entwicklung in der Implantologie. Ein besonderer Vorteil dieser Technik ist die zu erwartende vollkommene Körperverträglichkeit von Implantaten, die auf diese Art hergestellt werden. Da *ex vivo* gezüchtete Zellansammlungen zunächst weder die Form noch eine gewünschte mechanische Stabilität des späteren Implantats, wie Organe, Arterien, etc., aufweisen, werden solche Implantate zunächst auf einem formgebenden Substrat vorgeformt. Gängige Substrate, auf denen solche Zellen gezüchtet werden und die das primäre Stützgerüst für ein solches Implantat darstellen, sind beispielsweise Polylactide, Polyethylenglycole, Polyurethane, Teflon sowie anorganische Substrate.

Aus dem Stand der Technik sind weiterhin eine Vielzahl von Materialien bekannt und untersucht, welche für die Herstellung von derartigen primären Stützgerüsten bzw. Stützsubstraten Verwendung finden. So ist beispielsweise in der WO 98/56312 eine expandierbare Hülle aus ε-PTFE bekannt, die auch für die Züchtung von künstlichen Adern verwendet werden kann. Andere Materialien für diese Verwendung sind in der EP-A-0 810 845, US-Patent 4,883,699 und US-Patent 4,911,691 beschrieben. Weitere Polymere für den genannten Zweck sind beispielsweise hydrolysiertes Polyacrylnitril (US-Patent 4,480,642), hydrophile Polyether (US-Patent 4,798,876), Polyurethandiacrylate (US-Patent 4,424,395). Ferner sind verschiedene Hydrogele bekannt, die als Beschichtung für diesen Zweck eingesetzt werden können. Die Reihe der potentiell anwendbaren Materialien kann weiterhin ergänzt werden durch Polyvinylpyrrolidone (PVP), Polyvinylalkohole (PVA), Polyethylenoxid (PEO) und Polyhydroxyethylmethacrylate p(HEMA). Ferner werden im Stand der Technik auch die Anwendung einer Reihe von Standardmaterialien wie Polyurethane, Polyethylene und Polypropylene als mögliche Werkstoffe für derartige Substrate beschrieben. Ebenso sind Mischungen dieser Werkstoffe untereinander bekannt. Eine Reihe weiterer Materialien ist aus der EP-A-0 804 909 bekannt.

Da die inhärenten Eigenschaften dieser Materialien unterschiedlich sind, kann davon ausgegangen werden, daß jedes dieser Materialien bzw. jeder dieser Werkstoffe besondere Eigenschaften für bestimmte Anwendungen in der Züchtung von künstlichen Implantaten aufweist. So ist beispielsweise PVA sehr gut in Flüssigkeit löslich und wird schneller resorbiert. Andere Materialien weisen eine gute Blutverträglichkeit auf. Wiederum andere Materialien sind besonders gut streckbar. Jedoch weisen bedauerlicherweise alle Materialien Nachteile in unterschiedlichen Bereichen auf. PVA zeigt beispielsweise keine besonders gute Blutverträglichkeit.

ε-PTFE ist beispielsweise sehr gut streckbar und weist auch eine gute Blutverträglichkeit auf, jedoch ist dieses Material ausgesprochen schwierig zu handhaben und die Herstellung von Stützsubstraten aus diesem Material erfordert eine Reihe von bestimmten Verarbeitungsschritten (vgl. WO 96/00103). Auch ist die so gewonnene Oberfläche des ε-PTFE-Stützsubstrats sehr porös, so daß Zellen sehr stark in dieses Material hineinwachsen und beim Abtrennen des gezüchteten Zellmaterials als Implantat von dem Stützsubstrat eine Beschädigung fast unvermeidlich ist. Bei anderen Materialien können elastische Eigenschaften, die für ein derartiges Stützsubstrat in einigen Fällen wichtig sind, nur durch die Zugabe von Weichmachern erreicht werden, welche die Blut- und Körperverträglichkeit vermindern und durch Ausschwemmen der "Weichmacher" zusätzlich eine nachteilige Beeinflussung der Zellzüchtung darstellen.

Die größten bekannten Schwierigkeiten, die bei einer Züchtung von Zellen für Implantate auftreten, sind somit zum einen Reaktionen mit dem Stützsubstrat oder mit den hierbei entstehenden Abbauprodukten. So ist beispielsweise bekannt, daß bei der Auflösung bzw. der Resorption und der Zersetzung einiger der aus dem Stand der Technik bekannten Stoffe Entzündungsreaktionen im Empfänger auftreten können (van der Gießen, Circulation Band 94, Nr. 7, 1996). Dies ergibt sich entweder durch die teilweise mangelhafte Verträglichkeit solcher Stützsubstrate oder aber durch die Reaktion auf Zersetzungsprodukte, welche bei der Zersetzung der genannten Stoffe entstehen. Weiterhin können Risse und Brüche in dem frisch gezüchteten Implantat auftreten, wenn das gezüchtete Implantat von dem Stützsubstrat entfernt werden soll. Dieser nachteilige Effekt liegt insbesondere darin begründet, daß die für das Implantat wachsenden Zellen eine sehr enge Verbindung mit dem Stützsubstrat, insbesondere mit beispielsweise Polylactit, eingehen und durch die Porenstruktur, die entweder durch das Auflösen oder aber die grundsätzliche Oberflächenbeschaffenheit des Stützsubstrats entsteht, so mit dem Stützsubstrat verwachsen, daß ein Entfernen ohne Beschädigung des gezüchteten Implantats praktisch nicht möglich ist.

Weiterhin spielt für die problemfreie Züchtung der genannten Implantate, insbesondere Zellen, das Verhalten gegenüber Bakterien und Proteinen, die sich auf den Oberflächen der Stützsubstrate ablagern, eine zentrale Rolle, da diese Ablagerungen wesentlich zu Entzündungen im Patienten und anderen Problemen beim Wachstum und der Züchtung der Zellen führen können.

Insbesondere bei der Herstellung von Gefäßimplantaten sind die genannten Risse, die beim Entfernen des gezüchteten Gefäßimplantats vom Stützsubstrat entstehen können, ein wesentlicher Aspekt. Diese Risse sind beispielsweise Ansatzpunkte für eine verstärkte Thrombenbildung im Empfänger bzw. Patienten und andere Ablagerungen (Proteine, Makrophagen etc.), welche nach Implantation zu einem Risiko für den Empfänger bzw. Patienten werden können.

Somit liegt der vorliegenden Erfindung die Aufgabe zugrunde, ein neues System zur Herstellung von Implantaten aus biologischem Material bereitzustellen, welches ein möglichst selektives Wachstum von gewünschten Zellen ermöglichen soll sowie ein im wesentlichen beschädigungsfreies Abtrennen des aus den gewünschten Zellen gezüchteten Implantats vom eingesetzten Stützsubstrat sicherstellen soll.

Diese Aufgabe wird durch die in den Ansprüchen gekennzeichneten Ausführungsformen gelöst. Insbesondere wird die Verwendung eines Substrats mit einer formgebenden Oberfläche bereitgestellt, welche mindestens teilweise ein biokompatibles Polymer mit der folgenden allgemeinen Formel (I) 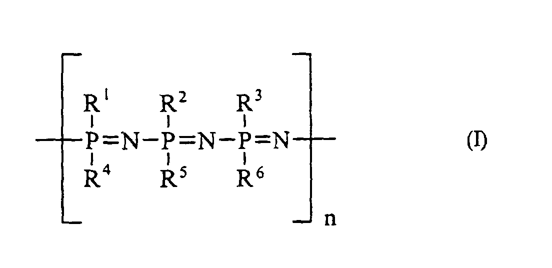 umfaßt, wobei n für 2 bis ∞ steht, R¹ bis R⁶ gleich oder unterschiedlich sind und einen Alkoxy-, Alkylsulfonyl-, Dialkylamino- oder Aryloxyreste oder einen Heterocycloalkyl- oder Heteroarylrest mit Stickstoff als Heteroatom bedeuten, als Matrize für die Züchtung gewünschter Zellen zur Herstellung von in einen Säuger implantierbarem biologischen Material.

In einer bevorzugten Ausführungsform der vorliegenden Erfindung wird das biokompatible Polymer gemäß Formel (I) als Überzug auf dem Substrat zur Ausbildung der formgebenden Oberfläche bereitgestellt. In dieser erfindungsgemäßen Ausführungsform weist das verwendete Substrat keine besondere Beschränkung auf und kann jedes Material, wie Kunststoffe, Metalle, Metallegierungen und Keramiken, sein. Der biokompatible Überzug weist beispielsweise eine Dicke von etwa 1 nm bis etwa 1000 µm, vorzugsweise bis etwa 10 µm und besonders bevorzugt bis etwa 1 µm auf. In einer anderen bevorzugten Ausführungsform der vorliegenden Erfindung ist das Substrat mit einer formgebenden Oberfläche ein Formkörper aus dem biokompatiblen Polymer gemäß Formel (I).

Der Polymerisationsgrad des biokompatiblen Polymers gemäß Formel (I) liegt vorzugsweise in einem Bereich von 20 bis 200.000, mehr bevorzugt von 40 bis 100.000.

Vorzugsweise ist mindestens einer der Reste R¹ bis R⁶ im verwendeten Polymer ein Alkoxyrest, der mit mindestens einem Fluoratom substituiert ist.

Die Alkylreste in den Alkoxy-, Alkylsulfonyl- und Dialkylaminoreste sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe. Beispiele für Alkylsulfonylreste sind Methyl-, Ethyl-, Propyl- und Butylsulfonylgruppen. Beispiele für Dialkylaminoreste sind Dimethyl-, Diethyl-, Dipropyl- und Dibutylaminogruppen.

Der Arylrest im Aryloxyrest ist beispielsweise eine Verbindung mit einem oder mehreren aromatischen Ringsystemen, wobei der Arylrest beispielsweise mit mindestens einem vorstehend definierten Alkylrest substituiert sein kann. Beispiele für Aryloxyreste sind Phenoxy- und Naphthoxygruppen und Derivate davon.

Der Heterocycloalkylrest ist beispielsweise ein 3- bis 7- Atome enthaltendes Ringsystem, wobei mindestens ein Ringatom ein Stickstoffatom ist. Der Heterocycloalkylrest kann beispielsweise mit mindestens einem vorstehend definierten Alkylrest substituiert sein. Beispiele für Heterocycloalkylreste sind Piperidinyl-, Piperazinyl-, Pyrrolidinyl- und Morpholinylgruppen und Derivate davon. Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem vorstehend definierten Alkylrest substituiert sein. Beispiele für Heteroarylreste sind Pyrrolyl-, Pyridinyl-, Pyridinolyl-, Isochinolinyl- und Chinolingruppen, und Derivate davon.

In einer weiteren bevorzugten erfindungsgemäßen Ausführungsform ist zwischen der Oberfläche des Substrats und dem aus dem Polyphosphazenderivat aufgebauten, biokompatiblen Überzug eine Schicht angeordnet, die einen Adhäsionspromotor enthält.

Der Adhäsionspromotor bzw. Spacer enthält bevorzugt eine polare Endgruppe. Beispiele hierfür sind Hydroxy-, Carboxy-, Carboxyl-, Amino oder Nitrogruppen. Es können aber auch Endgruppen des Typs O-ED verwendet werden, wobei O-ED einen Alkoxy-, Alkylsulfonyl-, Dialkylamino- oder Aryloxyrest oder einen Heterocycloalkyl- oder Heteroarylrest mit Stickstoff als Heteroatom bedeutet und unterschiedlich, beispielsweise durch Halogenatome, insbesondere Fluor, substituiert sein kann.

Insbesondere kann der Adhäsionspromotor beispielsweise eine Siliciumorganische Verbindung, vorzugsweise ein aminoterminiertes Silan bzw. basierend auf Aminosilan, aminoterminierte Alkene, nitroterminierte Alkene und Silane oder eine Alkylphosphonsäure sein. Besonders bevorzugt ist Aminopropyltrimethoxysilan.

Der Adhäsionspromotor verbessert insbesondere die Haftung des Überzugs auf der Oberfläche des Substrats durch Kopplung des Adhäsionspromotors an die Oberfläche des Substrats, beispielsweise über ionische und/oder kovalente Bindungen und durch weitere Kopplung des Adhäsionspromotors an das beschriebene Polymer der Formel (I) des Überzugs, beispielsweise über ionische und/oder kovalente Bindungen.

Der Begriff "biologisches Material" umfaßt beispielsweise eukaryontische Zellen, ein- oder mehrschichtige Zellverbände, Gewebe oder Zellbestandteile von Säuger-, insbesondere humanen Ursprungs. In einer bevorzugten Ausführungsform ist der Spender des biologischen Ausgangsmaterials identisch mit dem Empfänger für das implantierbare biologische Material. Beispiele für das biologische Ausgangsmaterial bzw. biologische Material sind Endothelzellen unterschiedlicher Herkunft (z.B. aus Haut, Vorhaut, Adern wie Aorta, Fettgewebe, Auge, Magennetz, Nabelschnur, Varizen oder ähnlichem), Epithelzellen unterschiedlicher Herkunft (z.B. aus Magen, Darm oder ähnlichem), Knochenzellen, Knorpelzellen, alle adhärenten Zellen oder Zellen, bei denen eine Adhärenz induzierbar ist, Zellverbände oder Gewebe (z.B. künstliche, in Kultur vorgezüchtete Haut oder ähnliche Gewebe), natürliche Gewebe sowie Proteine, Zuckermoleküle und Lipide. Unter Verwendung des Substrats mit formgebender Oberfläche können somit beispielsweise künstliche Organe, Adern, Knochen, Knorpel, Myelinscheiden etc. hergestellt werden.

## Patentansprüche

1. Verwendung eines Substrats mit einer formgebenden Oberfläche, welche mindestens teilweise ein biokompatibles Polymer mit der allgemeinen Formel (I) 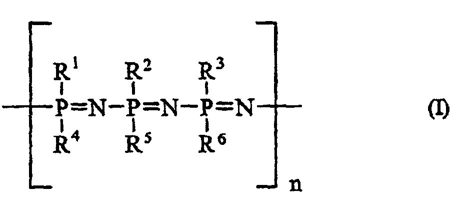 umfaßt, wobei n für 2 bis ∞ steht, R¹ bis R⁶ gleich oder unterschiedlich sind und einen Alkoxy- Alkylsulfonyl-, Dialkylamino- oder Aryloxyrest oder einen Heterocycloalkyl- oder Heteroarylrest mit Stickstoff als Heteroatom bedeuten, als Matrize für die Züchtung gewünschter Zellen zur Herstellung von in einen Säuger implantierbarem biologischen Material.

2. Verwendung nach Anspruch 1, wobei das biokompatible Polymer als Überzug auf der Substratoberfläche vorliegt.

3. Verwendung nach Anspruch 2, wobei zwischen der Oberfläche des Substrats und dem Überzug aus biokompatiblem Polymer eine Schicht angeordnet ist, die einen Adhäsionspromotor enthält.

4. Verwendung nach Anspruch 3, wobei der Adhäsionspromotor eine polare Endgruppe enthaltende Verbindung, insbesondere eine Silizium-organische Verbindung, ist.

5. Verwendung nach Anspruch 4, wobei die Silizium-organische Verbindung Aminopropyltrimethoxysilan ist.

6. Verwendung nach einem der Ansprüche 1 bis 5, wobei das implantierbare biologische Material Zellen, ein- oder mehrschichtige Zellenverbände, Gewebe oder Zellbestandteile umfaßt.

## Claims

1. Use of a substrate with a form-imparting surface which at least partially comprises a biocompatible polymer of the general formula (I) 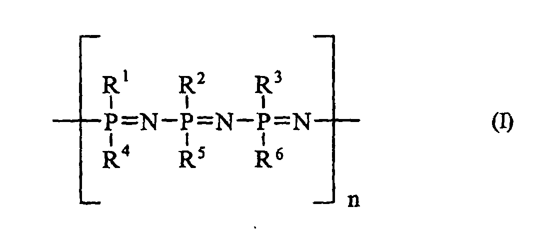 wherein n is 2 to ∞, R¹ to R⁶ are the same or different and mean an alkoxy, alkylsulfonyl, dialkylamino or aryloxy group or a heterocycloalkyl or heteroaryl group with nitrogen as heteroatom, as matrix for the cultivation of desired cells for the preparation of biological material implantable in a mammal.

2. The use according to claim 1, wherein the biocompatible polymer is present as a coating on the surface of the substrate.

3. The use according to claim 2, wherein a layer containing an adhesion promotor is provided between the surface of the substrate and the coating made of biocompatible polymer.

4. The use according to claim 3, wherein the adhesion promotor is a compound containing a polar terminal group, in particular a silicon-organic compound.

5. The use according to claim 4, wherein the silicon-organic compound is aminopropyltrimethoxysilane.

6. The use according to any one of claims 1 to 5, wherein the implantable biological material comprises cells, one- and multilayer unions of cells, tissue or cellular constituents.

## Revendications

1. Utilisation d'un substrat ayant une surface de façonnage, qui comprend au moins en partie un polymère biocompatible de formule générale (1) 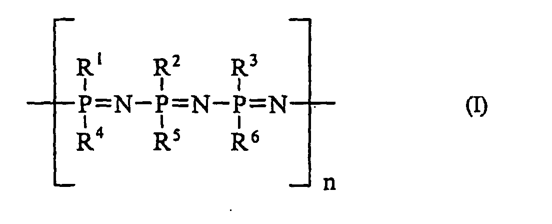 où n est 2 à ∞, R¹ à R⁶ sont identiques ou différents et représentent un résidu alcoxy, alkylsulfonyle, dialkylamino ou aryloxy ou un résidu hétérocycloalkyle ou hétéroaryle avec l'azote comme hétéroatome, comme matrice pour la culture de cellules souhaitées pour la fabrication d'un matériau biologique implantable chez le mammifère.

2. Utilisation selon la revendication 1, où le polymère biocompatible est présent sous forme de revêtement de la surface du substrat.

3. Utilisation selon la revendication 2, où, entre la surface du substrat et le revêtement en polymère biocompatible, une couche est placée qui contient un promoteur de l'adhérence.

4. Utilisation selon la revendication 3, dans laquelle le promoteur de l'adhérence est un composé contenant un groupe de queue polaire, en particulier un composé organosilicié.

5. Utilisation selon la revendication 4, dans laquelle le composé organosilicié est l'aminopropyltriméthoxysilane.

6. Utilisation selon une des revendications 1 à 5, dans laquelle le matériau biologique implantable comprend des cellules, des ensembles de cellules à une ou plusieurs couches, des tissus ou des composants cellulaires.
